(19) Europäisches Patentamt
European Patent Office
Office européen des brevets

(11) Veröffentlichungsnummer: **0 021 407**
**B1**

# (12) EUROPÄISCHE PATENTSCHRIFT

(45) Veröffentlichungstag der Patentschrift:
**05.12.84**

(51) Int. Cl.³: **G 01 N 33/52**, G 01 N 33/72, C 12 Q 1/28

(21) Anmeldenummer: **80103567.6**

(22) Anmeldetag: **24.06.80**

(54) **Verwendung substituierter oder kondensierter Pyridine in einem Verfahren zum Nachweis peroxidatisch wirksamer Substanzen, ein solches Verfahren und ein solche Pyridine enthaltendes Diagnostikum.**

(30) Priorität: **29.06.79 DE 2926271**

(43) Veröffentlichungstag der Anmeldung:
**07.01.81 Patentblatt 81/1**

(45) Bekanntmachung des Hinweises auf die Patenterteilung:
**05.12.84 Patentblatt 84/49**

(84) Benannte Vertragsstaaten:
**BE DE FR GB IT NL**

(56) Entgegenhaltungen:
**DE - A - 2 363 344**
**DE - A - 2 460 903**
**DE - A - 2 548 279**
**DE - A - 2 640 211**

**Die Akte enthält technische Angaben, die nach dem Eingang der Anmeldung eingereicht wurden und die nicht in dieser Patentschrift enthalten sind.**

(73) Patentinhaber: **BEHRINGWERKE AKTIENGESELLSCHAFT, Postfach 1140, D-3550 Marburg/Lahn (DE)**

(72) Erfinder: **Habenstein, Klaus, Dr., Birkenweg 3, D-3551 Lahntal (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 021 407 B1

ACTORUM AG

**Beschreibung**

Die Erfindung betrifft die Verwendung substituierter oder kondensierter Pyridine in einem Verfahren zum Nachweis peroxidativ wirksamer Substanzen und ein solches Verfahren, insbesondere ein Mittel zur Steigerung der Empfindlichkeit des Nachweises von peroxidativ wirksamen Substanzen, das vor allem verwendet wird in einem Diagnostikum bestehend aus einem Träger, einem Chromogen, einem Hydroperoxid, einem Stabilisator, einem Detergenz und einem Mittel zur Steigerung der Empfindlichkeit eines solchen Nachweises.

Der Nachweis peroxidativ wirksamer Substanzen, zu denen im tierischen Organismus Hämoglobin und Myoglobin gehören, besitzt eine erhebliche Bedeutung bei der Ermittlung kleiner, mit dem Auge nicht sichtbarer Mengen Blut in Körperflüssigkeiten wie Urin oder anderen Exkrementen, wie Stuhl oder Erbrochenem. Entscheidend für die Aussagekraft des Blutnachweises ist bei einer Mikrohämaturie neben der schnellen Verfügbarkeit des Ergebnisses eine hohe Empfindlichkeit. In Versuchen zur Erhöhung der Empfindlichkeit mittels aktivierender Zusätze zum Nachweissystem wurde bereits 1928 (Zschr. f. gerichtl. Med. 12, 216, 1928) erkannt, dass Chinolin, Isochinolin und Derivate von diesen den Nachweis beschleunigen.

Darauf aufbauend wurden später weitere Chinolin- und Isochinolinderivate geprüft und für gut befunden (DE-AS 1 242 905, DE-OS 2 640 211, DE-OS 2 548 279, DE-PS 2 235 152). Ebenso wurden bestimmte Pyridinderivate als gute Acceleratoren erkannt (DE-OS 2 363 344). Eine weitere Substanzklasse mit accelerierender Wirkung stellen Thiazol- und Benzothiazolderivate dar (DE-OS 2 652 545). Substanzen dieser Art werden insbesondere bei der Herstellung von Schnelldiagnostika (z.B. Teststreifen) eingesetzt.

Es wurde nun überraschenderweise gefunden, dass als Empfindlichkeitssteigerer ausser den bekannten Vinylpyridinen, Benzothiazolen oder Benzopyridinen im Prinzip alle Heteroaromaten mit wenigstens einem Stickstoffatom im Kern geeignet sind. Als neue Empfindlichkeitssteigerer mit guter Wirksamkeit erweisen sich dabei Phenyl-, Benzyl- und Benzoylpyridine. Wirksamkeit und Stabilität entsprechen denen der Vinylpyridine der DE-AS 2 363 344. Eine überraschende Steigerung von Stabilität und Wirksamkeit wurde gefunden, wenn eine zweite Verknüpfung zwischen dem Phenyl- und dem Pyridinkern in den genannten Verbindungen vorhanden ist.

Gegenstand der Erfindung ist die Verwendung einer Verbindung der chemischen Formel I

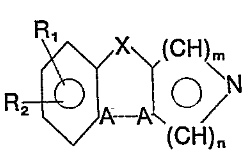

I

worin X $CH_2$, $CHR_1$, $CR_1R_2$, C=0 oder 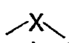 eine Bindung zwischen den beiden Kernen bedeutet, m und n eine ganze Zahl von 0 bis 3 sind, wobei m + n = 3 ist, und wenn X C=0 oder 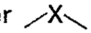 eine Bindung zwischen den beiden Kernen bedeutet, jedes A ein Wasserstoffatom ist, und, wenn X $CH_2$, $CHR_1$ oder $CR_1R_2$ ist, jedes A ein Wasserstoffatom ist oder beide –A zusammen eine Bindung zwischen den beiden Kernen bedeuten, und worin $R_1$ und $R_2$ Wasserstoff, Halogen oder Alkyl mit 1–4 Kohlenstoffatomen sind, oder ein Salz davon als Mittel zur Steigerung der Empfindlichkeit des Nachweises peroxidativ wirksamer Substanzen in einem Diagnostikum.

Gegenstand der Erfindung ist weiterhin ein Diagnostikum zum Nachweis peroxidativ wirksamer Substanzen, bestehend aus einem Träger, einem Chromogen, einem Hydroperoxid, einem Stabilisator, einem Detergenz und einem Mittel zur Steigerung der Empfindlichkeit des Nachweises, dadurch gekennzeichnet, dass das Mittel eine Verbindung mit der chemischen Formel I ist.

Bevorzugte Bedeutungen der Symbole sind:

| | |
|---|---|
| Für X: | $CH_2$. |
| Für $R_1$ und $R_2$: | Wasserstoff. |
| Für –A–––––A–: | eine Bindung. |

Besonders bevorzugt sind «Azafluorene» oder «Indenopyridine».

Das erfindungsgemässe Mittel wird bevorzugt in einem Diagnostikum eingesetzt, welches aus einem festen, streifenförmigen Trägermaterial, vorzugsweise aus Kunststoff, auf dem ein wasseraufnehmendes Testfeld vorhanden ist, besteht, das neben Puffersalzen, Hydroperoxid, Chromogen, Stabilisatoren und oberflächenaktiven Substanzen ein Mittel zur Steigerung der Empfindlichkeit der Formel I enthält.

Mit den erfindungsgemässen Empfindlichkeitssteigerern hergestellte Teststreifen reagieren sehr empfindlich auf kleinste Mengen Hämoglobin: Es können so noch Mikrohämaturien bis 0,03 mg Hämoglobin/l Urin (= 1–2 Erythrozyten/µl Urin) erkannt werden.

Durch Zusatz eines der bekannten Stabilisatoren (z.B. Hydrochinon, Ascorbinsäure) lässt sich die Stabilität des chemischen Systems bei Verminderung der Empfindlichkeit weiter erhöhen. Die dadurch erzielte Heraufsetzung der Anzeigenschwelle auf ca. 5 Erythrozyten/µl Urin ist bei Screening-Untersuchungen erwünscht, da im allgemeinen Mikrohämaturien erst als pathologisch angesehen werden, wenn mehr als 2–3 Erythrozyten pro µl vorliegen.

Als Puffer eignen sich beispielsweise Citrate, Phosphate oder Phthalate, die nach Anfeuchten des Testfeldes einen pH-Wert von 4–7, vorzugsweise 5–6 erzeugen.

Geeignete Hydroperoxide sind beispielsweise Cumol-, Tetralin-, Dekalin- oder Pinanhydroperoxid. Als Chromogene lassen sich beispielsweise Benzidinderivate, bevorzugt das nicht-kanzerogene Tetramethylbenzidin, verwenden.

Stabilisatoren vom Typ Ethylendiamino-tetraessigsäure schützen das Hydroperoxid durch Abfangen von Schwermetallspuren, Verdickungsmittel vom Typ Gelatine oder Polyvinylpyrrolidon stabilisieren das Chromogen und verhindern übermässiges Ausbluten des befeuchteten Testfeldes.

Beispiele weiterer Stabilisatoren sind aufgeführt in DE-PS 2 235 127, US-PS 4 071 317 oder 4 071 318. Detergenzien sind oberflächenaktive Substanzen wie beispielsweise Natriumdodecylsulfonat oder Dioctylnatriumsulfosuccinat.

Das wasseraufnehmende Testfeld kann grundsätzlich aus beliebigem Material bestehen. Verwendet werden allgemein Faservliese aus Cellulose oder Kunststoff, vorzugsweise Filterpapier. Es sind jedoch auch Systeme bekannt, in denen nichtfaserige Materialien verwendet werden, wobei die Chemikalien in einem wasseraufnehmenden Film enthalten sind.

Die folgenden Beispiele sollen die Erfindung erläutern:

Beispiel
Lösung 1
300 mg Polyvinylpyrrolidon,
10 mg EDTA-Di-Natriumsalz,
7,5 mg Tartrazin,
20 mg Dioctylsulfosuccinat-Na-Salz,
20 mg 4-Azafluoren,
2,2 mmol Zitronensäure und
30 mg 3,3',5,5'-Tetramethylbenzidin werden in
6 ml Methanol und
4 ml Wasser gelöst.
Der pH-Wert wird auf 5,5 eingestellt.
Lösung 2
60 mg Decalinhydroperoxid werden in
10 ml Toluol gelöst.

100 $cm^2$ Filterpapier werden mit Lösung 1 und nach Trocknung mit Lösung 2 getränkt.

Das Papier zeigt nach erneuter Trocknung eine Hämoglobinkonzentration von ca. 0,14 mg Hb/Liter Urin durch grüne Verfärbung deutlich an (entspricht etwa 5 Erythrozyten/µl).

Beispiel 2
300 mg Polyvinylpyrrodidon,
10 mg EDTA-Di-Natriumsalz,
7,5 mg Tartrazin,
20 mg Dioctylsulfosuccinat-Natriumsalz,
2,2 mmol Zitronensäure,
250 mg Cumolhydroperoxid,
30 mg 3,3',5,5'-Tetramethylbenzidin und
20 mg 4-Azafluoren werden in
6 ml Methanol und
4 ml Wasser gelöst.
Der pH-Wert wird auf 5,5 eingestellt.

100 $cm^2$ Filterpapier werden mit dieser Lösung getränkt und getrocknet.

Das fertige Papier gestattet, 0,03 mg Hb/Liter Urin (entspricht etwa 1–2 Erythrozyten/µl Urin) durch Grünfärbung des Papiers nachzuweisen. Dementsprechend werden 1–2 intakte Erythrozyten/µl Urin als deutliche grüne Pünktchen auf dem sonst gelben Untergrund angezeigt.

Wird das 4-Azafluoren durch 20 mg 2-Benzylpyridin, 2-Phenylpyridin oder 9-Brom-4-Azafluoren ersetzt, entspricht die Empfindlichkeit der Testpapiere der in Beispiel 1 angegebenen. Wenn es durch 20 mg 2-, 3- oder 4-Benzoylpyridin oder 3- oder 4-Benzylpyridin ersetzt wird, entspricht die Empfindlichkeit der Testpapiere einer Anzeige von 5 bis 10 Erythrozyten/µl Urin.

Beispiel 3
300 mg Polyvinylpyrrolidon,
10 mg EDTA-Di-Natriumsalz,
7,5 mg Tartrazin,
20 mg Dioctylsulfosuccinat-Natriumsalz,
2,2 mmol Zitronensäure,
250 mg Cumolhydroperoxid,
30 mg 3,3',5,5'-Tetramethylbenzidin und
20 mg 4-Azafluoren werden in
6 ml Methanol und einer Lösung aus
200 µg Ascorbinsäure in
4 ml $H_2O$ gelöst.
Der pH-Wert wird auf 5,5 eingestellt.

100 $cm^2$ Filterpapier werden mit dieser Lösung getränkt und getrocknet. Die Nachweisempfindlichkeit des fertigen Papiers ist vergleichbar mit derjenigen der Papiere des Beispiels 1.

## Patentansprüche

1. Verwendung einer Verbindung der chemischen Formel

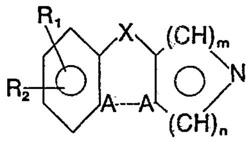

I

worin X $CH_2$, $CHR_1$, $CR_1R_2$, C=0 oder ⟋X⟍ eine Bindung zwischen den beiden Kernen bedeutet, m und n eine ganze Zahl von 0 bis 3 sind, wobei m + n = 3 ist, und wenn X C=0 oder ⟋X⟍ eine Bindung zwischen den beiden Kernen bedeutet, jedes A ein Wasserstoffatom ist, und, wenn X $CH_2$, $CHR_1$ oder $CR_1R_2$ ist, jedes A ein Wasserstoffatom ist oder beide –A zusammen eine Bindung zwischen den beiden Kernen bedeuten, und worin $R_1$ und $R_2$ Wasserstoff, Halogen oder Alkyl mit 1–4 Kohlenstoffatomen sind, oder ein Salz davon als Mittel zur Steigerung der Empfindlichkeit des Nachweises peroxidativ wirksamer Substanzen in einem Diagnostikum.

2. Diagnostikum zum Nachweis peroxidativ wirksamer Substanzen, bestehend aus einem Träger, einem Chromogen, einem Hydroperoxid, einem Stabilisator, einem Detergenz und einem Mittel zur Steigerung der Empfindlichkeit des Nachweises, dadurch gekennzeichnet, dass das Mittel eine Verbindung mit der chemischen Formel I gemäss Anspruch 1 ist.

## Claims

1. Use of a compound of the formula

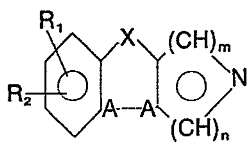

I

in which X is $CH_2$, $CHR_1$, $CR_1R_2$, C=0 or ⟋X⟍ represents a bond between the two nuclei, m and n represent an integer of from 0 to 3, m + n being 3, and if X stands for C=0 or ⟋X⟍ is a bond between the two nuclei, each A is a hydrogen atom, and if X is $CH_2$, $CHR_1$ or $CR_1R_2$ each A is hydrogen or both A together represent a bond between the two nuclei, and in which $R_1$ and $R_2$ are hydrogen, halogen or alkyl of from 1 to 4 carbon atoms or a salt thereof, in a diagnostic agent as a promotor of the sensitivity of the detection of substances having a peroxidatic effect.

2. Diagnostic agent for the detection of substances having a peroxidatic effect, which consists of a carrier, a chromogen, a hydroperoxide, a stabilizer, a detergent and a promotor of the sensitivity of the test, wherein the promotor is a substance with the formula I of claim 1.

**Revendications**

1. Utilisation d'un composé de formule chimique I

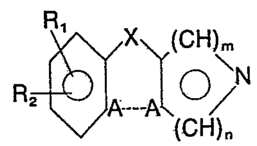

I

dans laquelle X est $CH_2$, $CHR_1$, $CR_1R_2$, C=0, ou ⟋X⟍ représente une liaison entre les deux noyaux, m et n sont des nombres entiers de 0 à 3, tels que m + n = 3, et lorsque X est C=0 ou ⟋X⟍ représente une liaison entre les deux noyaux, chaque A est un atome d'hydrogène, et lorsque X est $CH_2$, $CHR_1$ ou $CR_1R_2$, chaque A est un atome d'hydrogène ou deux A forment ensemble une liaison entre les deux noyaux, et dans laquelle $R_1$ et $R_2$ représentent l'hydrogène, un halogène ou un groupe alcoyle ayant de 1 à 4 atomes de carbone, ou un de ses sels, comme agent pour augmenter la sensibilité de la détection de substances à action peroxydante dans un agent de diagnostic.

2. Agent de diagnostic pour la détection de substances à action peroxydante, constitué d'un support, d'un chromogène, d'un hydroperoxyde, d'un stabilisant, d'un détergent et d'un agent pour augmenter la sensibilité de la détection, caractérisé en ce que l'agent est un composé répondant à la formule I selon la revendication 1.